# EUROPEAN PATENT APPLICATION

(11) **EP 4 796 033 A1**
(43) Date of publication of application: **26.08.2026**
(21) Application number: 25158920.6
(22) Date of filing: 19.02.2025
(51) Int. Cl.: A61B 5/15, B01L 3/00

(54) **A SAMPLE COLLECTION DEVICE**

(71) Applicant: True Dose AB, 171 65 Solna (SE)
(72) Inventor: RYDBERG, Per, 17165 Solna (SE); BROBERG, Fredrik, 17560 Järfälla (SE); NILSSON, Peter, 333 71 Bredaryd (SE); HEDAYATI, Elham, 17165 Solna (SE); HEDAYATI, Djavad, 17165 Solna (SE); HEDAYATI, Azita, 17165 Solna (SE); KOMNINOS, Nektarios, 17165 Solna (SE); DE CHIARA, Serena, 17165 Solna (SE)
(74) Representative: Potter Clarkson

(57) **Abstract**

The present disclosure provides a sample collection device, comprising: a well configured to receive a fluid sample, the well comprising a frangible base; a driving member configured to be moveable into the well and to isolate a predetermined volume of the fluid sample of that received within the well, the driving member further configured to move the predetermined volume of fluid sample out of an outlet of the sample collection device by breaking the frangible base of the well upon the application of a predetermined force and pushing the predetermined volume of the fluid sample through the outlet. There is also disclosed a kit of parts and method of using the sample collection device.

## Description

### Field

The present disclosure relates to a sample collection device, a kit of parts comprising a sample collection device and a sample vessel, and a method of isolating and outputting a predetermined volume of a fluid. In particular, the present disclosure is directed towards a sample collection device, associated kit of parts and associated method of isolating and outputting a predetermined volume of fluid. There is also disclosed herein a composition dispensing device, associated kit of parts and associated method of use.

### Background

Many clinical treatments necessitate the measurement of drug levels in bodily fluids, particularly in the context of therapeutic drug monitoring (TDM). TDM is essential for drugs with a narrow therapeutic window, where precise blood or plasma concentrations are critical for safety and efficacy. While commonly applied in the adjustment of dosages for various medications (e.g., anticonvulsants, anticoagulants), TDM practices are often underutilized for cytotoxic drugs used in cancer therapy, which have a high toxicity risk.

Phase I and II clinical trials primarily aim to establish pharmacokinetic (PK) profiles by determining half-lives and steady-state concentrations in plasma and urine. However, sampling over extended periods is complicated by drug degradation due to factors like exposure to oxygen and temperature changes, which can lead to inaccurate measurements if samples are not analysed promptly . As a result, the standard practice of adding internal standards during analysis may compromise the reliability of quantifications derived from delayed laboratory analyses.

Personalized medicine emphasizes tailoring drug dosages based on individual plasma levels, allowing clinicians to optimize treatment effectiveness and minimize adverse effects. Currently, accurate drug analysis typically requires clinical settings where samples are collected by professionals and sent to laboratories. The sample temperature combined with storage and transport time may cause unknown degradation already at 4°C.

There is a growing demand for portable devices that enable individuals to conduct self-sampling. Despite various solutions on the market, based on dried blood spot (DBS) technologies or short storage times, they are rarely used for therapeutic drug monitoring (TDM). However, DBS technology has several advantages relative to traditional blood sampling:
1. DBS is **minimally invasive,** requiring only a small finger-prick sample, which reduces patient discomfort and simplifies collection.
2. The samples are generally **stable and portable,** remaining stable at room temperature, lightweight, and easy to transport without cold-chain logistics.
3. Furthermore, DBS is **cost-effective,** with lower collection, storage, and shipping expenses compared to traditional methods.
4. It is **accessible** making it ideal for remote areas, self-collection, and large-scale studies .
5. Additionally, it requires **minimal blood volume,** making it suitable for neonatal and pediatric testing.

However, DBS technology also presents notable limitations that make it unsuitable for certain types of measurements. Challenges arise from uncertainties in sample volume, as the absorption of blood onto the DBS card is influenced by hematocrit levels and blood composition. While DBS can improve stability for certain analytes compared to traditional blood or plasma samples, degradation can still occur at variable rates, impacting accuracy. The most critical limitation is the low and inconsistent recoveries of analytes. Such systems can be impacted by significant deviations between actual blood concentrations and measured values.

Key areas where DBS technology struggles include:
1. **Accurate Plasma Drug Concentration:** For drugs that have significant differences in blood-to-plasma distribution, DBS cannot reliably replicate plasma drug concentrations, which is essential for TDM.
2. **Biomarkers in Very Low Concentrations:** The limited sample size can hinder the detection of analytes in trace amounts, necessitating more sensitive detection methods.
3. **Unstable Analytes:** Some analytes degrade quickly during storage, making DBS unreliable for unstable compounds.
4. **Comprehensive Blood Cell Analysis:** DBS does not preserve cellular components, limiting its use for analyses like complete blood count.
5. **Volume-Dependent Analyses:** Certain tests require larger sample volumes than what a single DBS spot can provide.
6. **Highly Time-Sensitive Biomarkers:** Delays during drying can lead to degradation of time-sensitive analytes.
7. **Hematocrit-Dependent Variations:** Variability in hematocrit affects blood spread on the DBS card, resulting in inaccuracies for certain analytes.

These challenges generally limit the use of portable sampling by showing poor coherence with traditional plasma analysis and highlight the need for alternative technologies. These challenges also include stability issues of measurement done in blood/plasma/serum that is not processed in immediate conjugation to the sampling.

### Summary

According to a first aspect of the present disclosure, there is provided a sample collection device, comprising: a well configured to receive a fluid sample, the well comprising a frangible base; a driving member configured to be moveable into the well and to isolate a predetermined volume of the fluid sample of that received within the well, the driving member further configured to move the predetermined volume of fluid sample out of an outlet of the sample collection device by breaking the frangible base of the well upon the application of a predetermined force and pushing the predetermined volume of the fluid sample through the outlet.

In one or more embodiments, the well may comprise an inlet arranged opposite the frangible base; the frangible base may comprise an engagement rod extending from the frangible base towards the well inlet; the driving member may be configured to engage with the engagement rod such that, upon engagement of the driving member with the engagement rod, the predetermined volume of the fluid sample is isolated; and the frangible base of the well may be configured to be broken upon the application of the predetermined force to the driving member when the driving member is engaged with the engagement rod.

In one or more embodiments, the driving member may comprise an end face and a socket arranged in the end face, the socket comprising a base which is set back from the end face of the driving member and wherein the engagement rod is configured to engage with the base of the socket.

In one or more embodiments, the length of the engagement rod may be such that, if a fluid collected within the well encapsulates a tip of the engagement rod, then there is sufficient fluid within the well to isolate the predetermined volume of fluid.

In one or more embodiments the sample collection device may comprise a composition chamber defined between the frangible base and the outlet of the sample collection device, wherein the composition chamber comprises a composition sealed therein, wherein the composition comprises one or both of: a fluid processing reagent and an internal standard and wherein the driving member is configured to push both the predetermined volume of the fluid sample and the composition through the composition chamber and out of the outlet.

In one or more embodiments, the composition chamber may be configured to protect the composition from degradation.

In one or more embodiments, the composition may comprise a substance that is configured to be used for the detection of sample loss of an analyte.

In one or more embodiments, the composition may be configured to function as a composition for quantification.

In one or more embodiments the sample collection device may further comprise an abrasive element stored within the composition chamber wherein the abrasive element is positioned such that when the driving member pushes the predetermined volume of the fluid sample and the composition through the outlet, the abrasive element is also pushed through the outlet and wherein the abrasive element is configured, to enhance mechanical mixing of the predetermined volume of fluid and the composition in a vessel into which they are pushed upon leaving the outlet.

In one or more embodiments, the abrasive element may be arranged within the composition chamber between the composition and the frangible base such that the composition is pushed out of the outlet by the engagement rod before the abrasive element.

In one or more embodiments the sample collection device may comprise a lower cap configured to close over the opening of a sample vessel, wherein the lower cap comprises the well.

In one or more embodiments, the lower cap may be configured to couple to a sample vessel by way of fastening threads on the lower cap that are configured to engage with complimentary fastening threads of a sample vessel.

In one or more embodiments the sample collection device may comprise an upper cap configured to close over the well inlet, wherein the upper cap comprises the driving member.

In one or more embodiments, the upper cap may comprise an upper cap body which is configured to be gripped by a user and wherein the upper cap is formed at least partially of material that allows inspection through the upper cap.

In one or more embodiments, the upper cap may comprise an upper cap body that is configured to be gripped by a use and wherein the upper cap body is rotationally isolated from the driving member such that rotation of the upper cap body does not result in rotation of the driving member.

In one or more embodiments, the upper cap may be configured to couple to the lower cap by way of complimentary fastening threads on the upper cap and the lower cap, wherein movement of the driving member through the well is controlled by the rotation of the upper cap relative to the lower cap and wherein the predetermined force is applied by the engagement rod by the rotation of the upper cap relative to the lower cap.

In one or more embodiments, the well may comprise a first volume having a first cross-sectional width and a second cross-sectional width, wherein the first cross-sectional width is smaller than the second cross-sectional width and wherein, when the driving member isolates the predetermined volume of fluid, the predetermined volume of fluid is contained within the first volume of the well.

In one or more embodiments, the may well comprise a channel arranged such that, as the driving member is moved through the well to isolate the predetermined volume of fluid, any excess fluid that would exceed the predetermined volume is displaced through the channel.

In one or more embodiments the channel may extend from an upper portion of the first volume to the second volume.

In one or more embodiments, the sample collection device may further comprise a seal arranged around a perimeter of the driving member, wherein the seal is configured to fluidly isolate the predetermined volume of fluid.

In one or more embodiments, the predetermined force required to break the frangible base may be greater than a force required to move the driving member through the well into a position which isolates the predetermined volume of fluid.

According to the second aspect of the present disclosure, there is disclosed a kit of parts comprising the sample collection device of the first aspect and a sample vessel in which the predetermined volume of fluid can be stored after it has moved out of the outlet of the sample collection device.

In one or more embodiments, the kit of parts may further comprise a lancet.

According to a third aspect of the present disclosure, there is disclosed a method of isolating and outputting a predetermined volume of a fluid, the method comprising: receiving fluid within a well of a sample collection device, the well comprising a frangible base; moving a driving member into the well, wherein the driving member is configured to isolate the predetermined volume of the fluid; applying a predetermined force to the driving member to break the frangible base of the well; moving the predetermined volume of fluid out of an outlet of the sample collection device by pushing the driving member to push the predetermined volume of fluid through the outlet.

According to a fourth aspect of the present disclosure, there is provided a composition dispensing device comprising a composition chamber wherein the composition chamber is sealed at a first end by a first frangible seal and at a second end by a second frangible seal, wherein the composition chamber comprises: a composition, wherein the composition is an internal standard of a fluid processing composition; and an abrasive element stored therein, wherein the abrasive element is arranged proximal to the first end of the composition chamber and the composition is arranged proximal to the second end of the composition chamber. The configuration and arrangement of the frangible seals, the abrasive element and the composition are such that a driving member which breaks the first frangible seal and moves through the composition chamber contacts the abrasive element and, upon the application of further force by a driving member, the abrasive element contacts the composition such that the abrasive member and the composition together break the second frangible seal and are forced out of the composition chamber. There is also disclosed a system comprising the composition dispensing device of the third aspect and a driving element configured to break the first frangible seal and push against the abrasive element in order to drive both the abrasive element and the composition out of the composition dispensing device through the second frangible seal.

While the disclosure is amenable to various modifications and alternative forms, specifics thereof have been shown by way of example in the drawings and will be described in detail. It should be understood, however, that other embodiments, beyond the particular embodiments described, are possible as well. All modifications, equivalents, and alternative embodiments falling within the spirit and scope of the appended claims are covered as well.

The above discussion is not intended to represent every example embodiment or every implementation within the scope of the current or future Claim sets. The figures and Detailed Description that follow also exemplify various example embodiments. Various example embodiments may be more completely understood in consideration of the following Detailed Description in connection with the accompanying Drawings.

### Brief Description of the Drawings

One or more embodiments will now be described by way of example only with reference to the accompanying drawings in which:
Figure 1 shows an example embodiment of a sample collection device of the present disclosure;
Figure 2 shows an example embodiment of a well in a first part of an example sample collection device;
Figure 3 shows an example cross-sectional view of a sample collection device of the present disclosure;
Figure 4 shows an example composition dispensing device according to an example of the present disclosure;
Figure 5 shows a kit of parts comprising the sample collection device of the present disclosure and a vessel; and
Figure 6 shows an example method of isolating and outputting a predetermined volume of fluid.

### Detailed Description

The present disclosure provides a sample collection device which is able to collect a fluid sample and output a predetermined volume of the fluid sample. By providing for fluid sample collection that is able to isolate and output a predetermined volume, the overall amount of fluid, such as blood, needed to be extracted from a patient is minimised while increasing the reliability of sample analysis. The sample collection device may further include the ability to dispense a composition along with the predetermined volume of fluid, where the composition comprises one or both of a fluid processing reagent, such as a blood processing reagent, and an internal standard. The composition may comprise a composition which is suitable for detecting, measuring or tracking the loss of an analyte in the fluid sample. By providing the ability to dispense the predetermined volume and a composition together, the sample collection device is able to provide for minimal, but sufficient, sample collection alongside the ability to track degradation of analytes within the sample in order to improve sample analysis.

In one or more embodiments of the present disclosure, to address the challenges associated with blood volume collection and to streamline the sampling procedure, the present disclosure provides a device that offers enhanced convenience and improved safety, significantly reducing the risk of exposure to chemicals. In one or more embodiments, the disclosed sample collection device is provided as a fully closed system that accurately extracts a required sample volume from an excess volume of blood.

Additionally, one or more embodiments of the disclosed sample collection device may be configured to process the collected sample internally, allowing for seamless mixing with internal standards and fluid processing reagents such as chemicals, enzymes, solutions, or other additives that can facilitate more effective analysis. This advancement not only simplifies the sampling process but also enhances the reliability and accuracy of test results, setting a new standard in blood sampling technology.

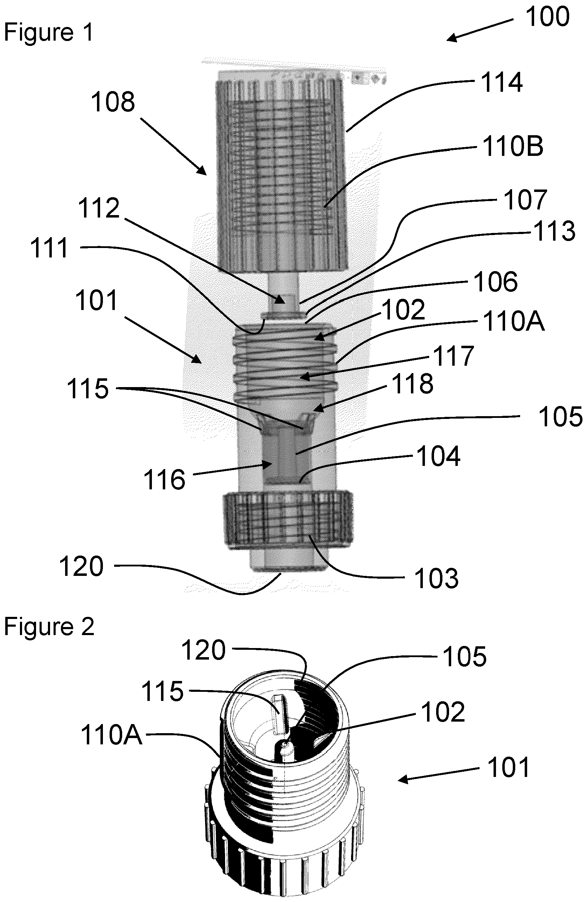

Figure 1 shows an example embodiment of a sample collection device 100 according to the present disclosure.

Figure 2 shows a view of an example embodiment of a lower part 101 of the sample collection device 100. Where figures in the present disclosure show corresponding features, the same reference numerals will be used between figures.

The sample collection device 100 comprises a well 102 configured to receive a fluid sample. The well 102 is appropriately sized to be able to receive at least a predetermined volume of fluid and, in one or more embodiments, the well 102 is sized to receive more than a predetermined volume of fluid. The well 102 may form part of a lower part 101 of the device 100. The lower part 101 of the sample collection device may be a lower cap 101 which is configured to close over the opening of a sample vessel (not shown), such as a sample vial or sample bottle. In embodiments where the lower part 101 of the sample collection device 100 is a lower cap 101 configured to close over the opening of a sample vessel, the lower cap 101 may be configured to couple to the sample vessel by way of fastening threads 103 on the lower cap that are configured to engage with complimentary fastening threads of the sample vessel. It will be appreciated that, in other embodiments, the lower part of the sample collection device may not need to close over a different sample vessel and may, instead, may be held over a sample vessel or other container into which the predetermined volume of fluid is to be dispensed when the predetermined volume of fluid is output from the sample collection device 100.

The well 102 comprises a frangible base 104. That is, the well 102 comprises a base 104 which is configured to be broken by the application of a predetermined force. In particular, the frangible base 104 comprises one or more features which makes it suitable to be broken in a controlled manner by the application of at least the predetermined force. The features which provide for the base 104 to be frangible may include one or more points of weakness which allow for the base to be separated from the remainder of the well 102 or any other suitable features which would allow for the base 104 to be configured to be breakable. In other embodiments, the frangible base 104 may comprise a hinge or other opening mechanism which can only be opened by way of the application of the predetermined force. In one or more embodiments, the frangible base 104 may be configured to completely detach from the remainder of the well 102 upon the application of the predetermined force. In other embodiments, the frangible base 104 may be configured to stay connected to the remainder of the well 102, at least in part, upon the application of the predetermined force.

The well 102 may comprise an engagement rod 105 that extends from the frangible base 104 towards the inlet 106 of the well. That is, the engagement rod 105 may extend through the well 102 from the base 104. The length of the engagement rod 105 may be such that, if a fluid sample collected within the well 102 encapsulates a tip of the engagement rod 105, then there is a sufficient fluid within the well 102 to isolate the predetermined volume of fluid. The length of the engagement rod 105 may be such that, if the fluid perfectly encapsulates the tip of the engagement rod 105, then the well 102 comprises exactly the predetermined volume of fluid for isolation. In other embodiments, the length of the engagement rod 105 may be such that, if the fluid perfectly encapsulates the tip of the engagement rod 105, then the well 102 comprises more than the predetermined volume of fluid so that the predetermined volume can be isolated from the remaining (excess) volume of fluid. Either configuration may help to ensure that the minimum predetermined volume is isolated. It will be appreciated that, where a discussion is provided of fluid covering the tip of the engagement rod 105, that this is based on the base 104 of the well 102 being aligned substantially parallel with flat ground such that the fluid does not extend higher up one side of the well 102 than another side of the well 102. The engagement rod 105 may provide an addition benefit in that, as droplets of fluid enter the well, they are likely to come into contact with the engagement rod 105. The droplets of fluid are then guided by the engagement rod 105 into the well. As a result of this, providing the engagement rod 105 reduces the chances of air entrapment in the droplet, thereby preventing the formation and entrapment of air bubbles. Air entrapment in a droplet of fluid may prevent the correct volume from being reached.

In the context of the present disclosure, it will be appreciated that the isolation of the predetermined volume of fluid means that the predetermined volume of fluid is suitably contained within a sub-volume of the sample collection device 100. This may cause the predetermined volume of fluid to be isolated, for example, from an excess volume of fluid which is diverted to a different part of the sample collection device 100. or out of the sample collection device 100. In other examples, the predetermined volume of fluid is isolated only from the external environment, as an excess volume of fluid may not be present. Further, it will be understood that isolation in this case refers to isolation of the sample fluid from escape from the sub-volume in which it is trapped. For example, where the fluid is blood, the sample collection device 100 may be configured to prevent the blood from escaping the sub-volume in which it is contained upon isolation. In some embodiments, the isolation may not provide isolation to gasses but only to fluids. In other examples, the isolation may be provided by way of a hermetic seal which provides for isolation to both liquid and gasses.

Further, the fluid sample which is to be isolated may be any suitable fluid sample. In one or more embodiments, the fluid sample may be a bodily fluid sample which may include, for example, blood, urine, spinal fluid, saliva, sweat, semen or any other suitable fluid. It will be appreciated that the sample collection device of the present disclosure may also be used to collect non-bodily fluid fluids.

The sample collection device 100 further comprises a driving member 107 configured to be moveable into the well 102 and to isolate a predetermined volume of the fluid sample received within the well 102. In one or more embodiments, the driving member 107 may comprise part of an upper part 108 of the sample collection device 100. The upper part 108 of the sample collection device 100 may be an upper cap 108 which is configured to close over the well 102 such that, when correctly placed, an inlet 106 of the well 102 is closed in order to prevent the egress of sample fluid from the well inlet 106. In embodiments wherein the upper part 108 is an upper cap 108, the upper cap 108 may be configured to couple to the lower part 101 (which may be a lower cap 101) by way of complimentary fastening threads 110A, 110B on the upper cap 108 and the lower cap 101. The movement of the driving member 107 through the well 102 may be controlled by the rotation of the upper cap 108 relative to the lower cap 101. As a result of the complimentary fastening threads 110A, 110B, the predetermined force applied by the driving member 107 may be applied by the rotation of the upper cap 108 relative to the lower cap 101.

In embodiments which comprise the engagement rod 105, the driving member 107 may be configured to engage with the engagement rod 105 such that, upon engagement of the driving member 107 with the engagement rod 105, the predetermined volume of fluid sample is isolated. The engagement of the driving member 107 and the engagement rod 105 may provide for a visually clear indication to a user that isolation of the predetermined volume of fluid has been achieved. Further, the frangible base 104 of the well 102 may be configured to be broken upon the application of the predetermined force to the driving member 107 when the driving member 107 is engaged with the engagement rod 105. That is, the predetermined force may be able to be transferred from the driving member 107 to the frangible base 104 via the engagement rod 105. This may provide for a controlled and targeted way of ensuring that the predetermined force is applied at the correct point. In other embodiments, however, the driving member 107 may contact the frangible base 104 or other part well 102 to provide for the transfer of the predetermined force to the frangible base 104 without the need for an engagement rod 105.

In one or more embodiments, the driving member 107 may comprise an end face 111 and a socket 112 arranged in the end face 111. The socket 112 may comprise a base which is set back from the end face 111 of the driving member 107 and the engagement rod 105 may be configured to engage with the base of the socket 112. The socket 112 in the end face 111 of the driving member 107 may provide a convenient way to ensure that the engagement rod 105 does not slip or otherwise disengage from the driving rod 107. The socket 112 may also allow for any seal 113, discussed later, which provides for or enhances isolation of the fluid, to be moveable slightly below the top of the engagement rod 105. This may be advantageous for isolating the predetermined volume of fluid sample from an excess volume of fluid sample in embodiments where the length of the engagement rod 105 provides for an indication of when a volume of fluid collected is greater than the predetermined volume of fluid for isoaltion.

The upper part 108 (which may be an upper cap 108) of the sample collection device 100 may be configured to be gripped by the user so that the predetermined force can be applied by the user. While the complimentary screw threads 110A, 110B have already been explained as one way in which the upper part 108 may be configured to apply the predetermined force to the frangible base 104, it will be appreciated that other approaches may exist. For example, the upper part 108 may be configured to apply force by one or more of: of a ratcheted mechanism; the downward application of force by a user without additional mechanisms; a threaded fastening; or any other suitable method.

In one or more embodiments, the upper part 108 may comprise a cap body 114 which is configured to be gripped by a user. The cap body may be, for example, a handle. The upper cap body 114 may be rotationally isolated from the driving member 107 such that rotation of the upper cap body 114 does not result in rotation of the driving member 107.

The cap body 114 of the sample collection device 100 may be formed at least partially of a material that allows for inspection through the cap body 114. This may be particularly beneficial when the predetermined sample volume has been, or has almost been, isolated, as it may allow a user to inspect whether the predetermined volume has been isolated properly or not. If the predetermined volume has not been isolated, the user knows that they should apply further force. The cap body 114 of the sample collection device 100 may be at least in part, for example, diaphanous, which is to say that the cap body 114 may be at least in part either transparent or translucent.

The driving member 107 may be configured to isolate the predetermined volume of the fluid sample by way of the complimentary configuration of the driving member 107 and the well 102. For example, the driving member 107 may be shaped such that it engages with sides of the well 102 at a particular point within the well 102 in order to isolate the predetermined volume of fluid. In one or more embodiments, one or both of the driving member 107 and the well 102 may comprise a seal 113 that is configured to fluidly isolate the predetermined volume of fluid. For example, the seal 113 may be an O-ring or other feature which provides for, or enhances, the sealing effect of the engagement of the driving member 107 with the edges of the well 102. For example, the seal 113 may be a cup seal, a lip seal or a bellows seal. The seal 113 may be arranged, for example, around a perimeter of the driving member 107. In some embodiments, the seal 113 may be arranged around a perimeter of the end face 111 of the driving member 107. The seal 113 may be configured to provide for or enhance the fluid isolation of the predetermined volume of fluid.

The well 102 may comprise one or more features which ensure that no more than the predetermined volume of sample fluid is isolated. For example, the well 102 may comprise a channel 115 arranged such that, as the driving member 107 is moved through the well 102 to isolate the predetermined volume of fluid, any excess fluid that would exceed the predetermined volume is displaced through the channel 115. This channel 115 may be implemented, for example, by having a slot-shaped channel 115 extending from a point just above the height in the well 102 where the predetermined volume would be stored so that, as the driving member 107 moves through the well 102, excess fluid is moved through the channel 115 until the driving member 107 reaches a point within the well 102 that isolates the channel 115 from the predetermined volume within the well 102. In one or more embodiments, there may be provided a plurality of such channels 115.

In one or more embodiments, the well 102 may comprise a first volume 116 having a first cross-sectional width and a second volume 117 having a second cross-sectional width different to the first cross-sectional width. In particular, the first cross-sectional width may be smaller than the second cross-sectional width. The driving member 107 and the well 102 may be configured, by their relative shapes and sealing features, to isolate the predetermined volume of fluid within at least part of, and optionally the whole of, the first volume 116 of the well 102. The first volume 116 and the second volume 117 may be separated by a transitional volume 118, the cross-sectional width of which may transition from the first cross-sectional width to the second cross-sectional width. The transition from the first cross-sectional width to the second cross-sectional width may occur over a non-zero distance such that a steady transition is observed. In other embodiments, the second volume 117 may be considered to be the volume which comprises the part having the second cross-sectional width and the portion of the well which transitions from the first cross-sectional width to the second cross-sectional width. In one or more embodiments, the channel 115 or channels 115 discussed above which provide for the movement of excess fluid out of the predetermined volume may extend from an upper portion of the first volume 116 to one or both of the second volume 117 and the transitional volume 118.

Returning to the driving member 107, the driving member 107 may be configured to move the predetermined volume of fluid out of an outlet 120 of the sample collection device 100 by breaking the frangible base 104 of the well 102 upon the application of the predetermined force and pushing the predetermined volume through the outlet 120. This may allow the sample collection device 100 to not only collect and isolate a predetermined volume of fluid, but also to dispense the desired volume into another vessel or volume of some sort. This may allow the fluid sample to be transported to, for example, a laboratory for testing, prior to dispensing. In other embodiments, this may allow the predetermined volume to be isolated and dispensed rapidly prior to analysis or alternative storage in another sample vessel.

In one or more embodiments, the predetermined force required to break the frangible base 104 may be greater than a force required to move the driving member 107 through the well 102 into a position which isolates the predetermined volume of fluid. That is, a greater resistance may be encountered by a user once the predetermined volume of fluid has been isolated. This may allow the user to easily understand when the predetermined volume of fluid has been isolated. This may be particularly advantageous, as it may allow the user to stop at this point and keep the sample isolated prior to dispensing. This may provide for a storage mode in which the predetermined volume of fluid is stored within the sample collection device.

In one or more embodiments, the upper and lower parts 108, 101 may be rotated until two printed arrows or other indicia on the device align, with physical resistance temporarily halting the rotation. Once aligned, further rotation can continue to break the frangible base 104.

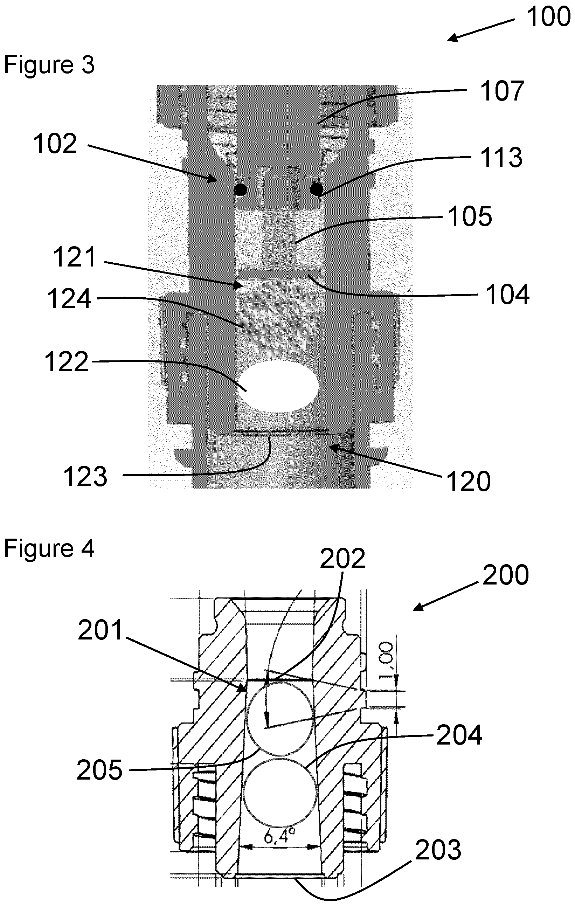

Figure 3 shows an example embodiment of the sample collection device 100 when the driving member 107 is engaged within the well 102 such that the predetermined volume of fluid sample is isolated.

Figure 3 further shows an embodiment where the sample collection device 100 comprises a composition chamber 121 defined between the frangible base 104 and the outlet 120 of the sample collection device 100. The composition chamber 121 comprises a composition 122 sealed therein. The driving member 107 may be configured to push both the predetermined volume of the fluid sample and the composition 122 through the composition chamber 121 and out of the outlet 120. In one or more examples, the frangible base 104 may be located at a first end of the composition chamber 120. The second end of the composition chamber 120 may be defined, in part or fully, by a frangible barrier 123 which is configured to suitably seal the composition within the composition chamber 121 but be breakable so that the composition 122 and predetermined volume of fluid can be pushed therethrough. The frangible barrier 123 may be any suitable barrier, such as: a plastic barrier having weak-points defined in its coupling to the edges of the composition chamber 121; a film barrier which is broken by the application of pressure thereto or any other suitable barrier including, but not limited to, the options presented above with respect to the frangible base 104. The frangible barrier 123 has the added flexibility when compared to some embodiments of the frangible base 104 that it does not have an engagement rod 105 that extends therefrom and so it may be formed of a more flexible or otherwise delicate material.

The composition 122 comprises one or both of a fluid processing reagent and an internal standard.

A fluid processing reagent, which may be a blood processing reagent, may be any suitable chemical, solution, enzyme or other additives appropriate for processing biological fluids. It will be appreciated that a plurality of different blood processing reagents may be included as part of the composition.

By the term Internal Standard in the context of chemical analyses, we refer to compounds that are used for the detection of sample losses of the analyte, e.g. the analyte in the sample, to be determined. Internal Standards are known substances with similar analytical behaviour to the substance to be evaluated (i.e. the target compounds/moieties present in a sample), e.g. a stable or analogue isotope of the analyte. Internal Standards may also be, for example, stable isotope substituted chemicals, or near identical, to the chemicals being measured in the sample. In contrast to external standards, these substances are located in the sample and are "also treated" - thus internal. In chromatographic bioanalysis for example, an Internal Standard is added to all samples, including calibration standards, as well as quality controls and samples before extraction. Therefore, Internal Standards serve as a reference for analysing and quantifying the presence of target compounds/moieties present in a sample.

The composition 122 comprising the substance suitable for use as an Internal Standard may be a matrix composition 122. The purpose of the matrix is to absorb the Internal Standard and to release the standard to the solution simultaneously with the biological sample by the catch and release principle. The purpose with the matrix is also to protect the analyte from un-desired by reactions such as solvolysis, reaction between solvents in the tube or atmosphere (water, alcohol or other reactive solvents).

The matrix composition 122 can comprise, for example, a reverse phase silica stationary phase or a polymeric stationary phase that retains the Internal Standard(s) by hydrophobic interactions delivered from the incorporated aliphatic groups (C1-C18 chains, phenyl, diphenyl or other functional groups used within the art of liquid chromatography). These matrix compositions 122 may also or alternatively comprise a mixed-mode stationary phase, reversed silica or a polymeric phase, modified with polar groups such as aromatic or aliphatic hydroxyl groups, aromatic or aliphatic amines or other groups that retain the Internal Standard by both hydrophobic interactions and polar interactions, with anionic interactions and cationic interactions.

The matrix composition 122 may also or alternatively comprise a reversed phase silica gel or a polymer (e.g., polypropylene, polyethylene, polyamide etc., including non-woven polypropylene, preferably polypropylene, polyethylene, polyamide etc.) which absorbs the Internal Standard. The matrix composition 122 may also comprise a cosolvent (e.g., Pure C8 MTC oil, mineral oil, dimethyl sulfoxide (DMSO), dimethyl formamide (DMF), natural oils and methyl esters of their constitutive fatty acid such as rapeseed methyl ester and others), mixtures solvents given above with of aqueous pH-adjusted buffers (HEPES, phosphate etc.) preferably added in small amounts to increase the release reaction rate and increase the solubility of a poorly soluble compound. The matrix composition 122 may also or alternatively comprise a solid phase that is soluble in any solution, such as an analysis/extraction solution, used in the tube (e.g., protein precipitation solutions, anticoagulants or other solvent mixtures employed to preserve the analytes in biological fluids and/or facilitate the analysis). For example, the composition 122 may comprise excipients into the matrix of which the substance suitable for use as an Internal Standard may be incorporated. The matrix composition 122 may further comprise a solid (e.g. a solid made of tert-butanol, carboxymethylcellulose, Arabic gum, coconut wax etc.) containing the Internal Standard but being soluble in the solvents used for protein precipitation (i.e. the solvents in the analysis/extraction solution as defined below). To further increase the stability of the Internal Standard absorbed to a matrix, scavengers, such as antioxidants (quercetin, vitamin-E, beta carotene, 2 6-diisopropylphenol) can be comprised in the composition 122 to further protect the Internal Standard. Such anti-oxidants may be selected from the list consisting of vitamin-E (Tocopherol), beta carotene, 2 6-diisopropylphenol, butylated hydroxyanisole (BHA), butylated hydroxytoluene (BHT) and ascorbic acid (vitamin C).

When incorporating antioxidants into the composition 122, such anti-oxidants may be sterically hindered. For example, they may be configured to be sterically hindered so as to not interact with components in the matrix that comprise oxidising groups, but are still able to act as anti-oxidants against free oxygen and reactive oxygen species (ROS) that are generated/introduced during storage. Suitable sterically hindered anti-oxidants include butylated hydroxytoluene (BHT), and butylated hydroxyanisole (BHA).

Preferably the matrix composition 122 comprises a non-woven polypropylene fabric, which may be a pompom ball (diameter 5 mm, or any other suitable size) or a plain non-woven polypropylene fabric which may have a thickness of from about 0.05 mm to about 0.5 mm, such as from about 0.1 mm to about 0.3 mm.

The matrix composition 122 may comprise silica particles onto which the substance suitable for use as an Internal Standard may be adsorbed such that on entry into the sample vessel interior the substance may be released from the surface of the silica particles.

The composition 122 may further comprise indicator compound.

The term "indicator compound" should be understood to include any chemical compound that causes a change of colour (i.e. visual change) when added to the analysis/extraction solution, for example a chemical compound that causes the colour of the analysis/extraction solution to change upon release of the composition 122 into the interior of the sample vessel, and mixing of the indicator (i.e. the composition 122) with the analysis/extraction solution. In this way, the indicator compound may act as a visualisation aid, and allow the user to visually monitor the activation process as the colour of the analysis/extraction solution changes following addition of the composition 122. Furthermore, the indicator compound may provide for visual identification of early, unintentional or accidental release of the composition 122 into the analysis/extraction solution, for example in the case where the bottom seal is damaged prior to sampling (e.g. during storage or transport).

The indicator compound may further act as an antioxidant. By "antioxidant", this may be understood as comprising means to minimise oxidation of the Internal Standard, and thereby protect the Internal Standard from excessive oxidation so as to extend the shelf-life of the apparatus, sample vessel and/or kit-of-parts. The addition of antioxidants to the standard will, in addition to protect the standard, also protect the collected blood sample, including its components, from oxidation.

The indicator compound may be any suitable dye compound or colorant. Suitable indicator compounds that may been included may be selected from one or more of triarylmethane basic dyes, flavin basic dyes, auramine basic dyes, safranine basic dyes, Phloxine basic dyes, xanthene basic dyes and methylene blue bases.

Further suitable indicator compounds may be selected from one or more of Sudan Blue II, Methyl Violet, Crystal Violet, Magenta, Basic Cyanine 6G, Basic Cyanine EX, Victoria Pure Blue BO, Victoria Blue B conc., Brilliant Green GX, Malachite Green, Basic Yellow 1, Basic Red 2, Basic Red 12, Basic Blue GO, New Methylene Blue NX, Methyl Viologen, Methyl Red, Methylene Blue, Methyl Orange, tris(bipyridine)ruthenium(II) chloride, Rhodamine B, Rose Bengal, Brilliant Blue, Indigo Carmine, Patent Blue, Direct Yellow, Serva Blue, Prussian Blue, triarylmethane, flavine, xanthene, bikaverin, fluorescein and eosin. Preferably, the indicator compound is selected from one or more of Blue II, Methylene Blue and Rhodamine B. More preferably, the indicator compound is Sudan Blue II.

The indicator compound may be included in the composition 122 in an amount of from about 0.001 wt.% to about 0.1 wt.%, such as from about 0.001 wt.% to about 0.05 wt. %, for example from about 0.001 wt.% to about 0.01 wt.%.

The Internal Standard may be included in the composition 122 in an amount of from about 0.001 wt.% to about 0.05 wt. %, for example from about 0.001 wt.% to about 0.01 wt.%.

The Internal Standard may be included in the composition 122 in an amount of from about 0.001 wt.% to about 0.1 wt.%, such as from about 0.001 wt.% to about 0.05 wt. %, for example from about 0.001 wt.% to about 0.01 wt.%.

The composition 122 may further comprise one or more radical inhibitor or one or more oxygen radical inhibitor. The terms "oxygen radical inhibitor" and "radical inhibitor" should be understood to include any chemical compound that prevents the formation of free radicals from oxygen or others, and/or terminates radical chain reactions, thereby stabilizing materials within the composition 122.

Suitable radical inhibitors that may be included may be selected from the list consisting of hydroquinone, tertiary butylhydroquinone (TBHQ), phenols, polyphenols, gallic acid, naringin and quercetin.

The composition 122 may further comprise one or more pH modifier. The term "pH modifier" should be understood to include any chemical compound that adjusts the acidity or alkalinity of a solution to stabilize sensitive chemicals that are prone to degradation in certain pH environments. In this context, the pH modifier acts to modify the solution in the sample vessel when the composition 122 is introduced into the sample vessel.

Suitable pH modifiers that may be included may be selected from the list consisting of citric acid, formic acid, acetic acid, sodium hydroxide, hydrochloric acid, HEPES, TRIS (tris(hydroxymethyl)aminomethane) and similar buffers.

The composition 122 may further comprise one or more scavengers. The term "scavenger" should be understood to include any chemical compound that reacts with and/or neutralizes reaction species within the composition 122, such as oxygen, peroxides or metal ions that could catalyse degradation reactions.

Suitable scavengers that may be included may be selected from the list consisting of ethylenediaminetetraacetic acid (EDTA), activated charcoal and sodium bisulfite.

The composition 122 may further comprise one or more stabilising agents. The term "stabilising agent" should be understood to include any chemical compound that aids in maintaining the integrity and functionality of the chemicals and materials within the composition 122 over time by preventing degradation through various mechanisms.

Suitable stabilising agents that may be included may be selected from the list consisting of polyvinylpyrrolidone (PVP) and glycerol.

The composition 122 may further comprise one or more preservatives. The term "preservatives" should be understood to include any chemical compound that prevents microbial growth (e.g., bacteria growth, fungi growth and other microorganism growth) that could potentially lead to degradation of materials within the composition 122. The term "preservative" also encompasses antimicrobial agents.

Suitable preservatives/antimicrobial agents that may be included may be selected from the list consisting of sodium benzoate, parabens (e.g., methylparaben and propylparaben), sorbic acid, silver ions, triclosan and chlorhexidine.

The composition 122 may further comprise one or more humectants. The term "humectant" should be understood to include any chemical compound that retains moisture to prevent drying out and degradation of products.

Suitable humectants that may be included may be selected from the list consisting of glycerin, 2,5-hexanediol.

The composition 122 may be a dry pellet and may also be shaped so as to enable the dry pellet to penetrate the films on the application of pressure. For example, the end of the dry pellet that is towards the lower penetrable film may be shaped to a tapered point, such that when the sampler, or the rod member of the lid, is passed through the aperture of the end cap this places pressure on the top of the dry pellet and, with the bottom end of the pellet being tapered, this pierces the lower film causing the pellet to enter the interior of the sample vessel.

In one or more embodiments, it may be desirable for a user to apply the predetermined force to cause the frangible base 104 to be broken and, thereby, for the predetermined volume of fluid to be forced through the previous location of the frangible base 104 and into the composition chamber 121. In one or more embodiments, the user may not initially continue to apply force in order to cause the predetermined volume of fluid to be forced through the frangible barrier 123 and out of the outlet 120 of the sample collection device 100. One or more features of the sample collection device 100 may facilitate the user's transfer of the predetermined volume of fluid into the composition chamber 121 without dispensing it from the sample collection device 100 entirely. For example, placement and configuration of the frangible base 104, the composition 122 and the frangible barrier 123 may be such that the user is able to notice when the frangible barrier is initially broken, thereby indicating to the user that they should not apply additional force until they want to dispense the predetermined volume of fluid from the composition chamber. Additionally or alternatively, the sample collection device may comprise one or more visual indicia which are configured to inform the user when they have successfully broken the frangible base. Yet further visual indica may be provided to indicate to the user the point at which additional movement of the driving member 107 will result pushing the predetermined volume of fluid through the frangible barrier 123 and out of the outlet 120.

By providing means for a user to be able to selectively force the predetermined volume of fluid into a composition chamber 121 which comprises a composition 122 which comprises fluid processing reagent and/or an internal standard, the user is able to initiate one or both of a processing or standardisation mode in which the fluid begins being processed and/or wherein the internal standard can be used to track degradation of one or more analytes.

In one or more embodiments, the frangible barrier 123 may be configured to deteriorate over a predetermined period of time. The planned deterioration of the frangible barrier 123 may allow for the predetermined volume of fluid stored in the composition chamber 121 to be accurately released after the predetermined amount of time. This may be particularly beneficial in embodiments where the composition 122 comprises one or more fluid processing reagents, as it may provide for a desired and reliable amount of processing prior to release. In embodiments in which the frangible barrier 123 is configured to deteriorate, the deterioration may be provided by the frangible barrier 123 being a water-soluble barrier configured to dissolve after a predetermined amount of time. Examples of suitable plastic materials that are soluble in water include certain types of cellulose derivatives, such as: Polyvinyl Alcohol (PVA): A water-soluble synthetic polymer that is resistant to organic solvents; Hydroxypropyl Methylcellulose (HPMC): A modified cellulose that dissolves in water but remains unaffected by alcohols and most organic solvents; and Gelatin: A natural polymer that is soluble in water but insoluble in alcohol. It may be particularly desirable that these frangible barrier materials are insoluble in alcohol.

In one or more examples, the internal standard may serve a dual purpose: it may be designed to detect sample loss of an analyte due to degradation during transport, storage, and analytical processing, and it may further function as an internal standard for quantification

The composition chamber 121 may be configured to protect the composition 122 from degradation. This protection from degradation may be achieved in any of a plurality of ways. For example, the composition chamber 121 may comprise a vacuum such that degradation of the composition 122 by way of interaction with the air does not occur. In other examples, the composition chamber 121 may be filled with a suitable inert gas which prevents or lessens degradation of the composition 122. In one or more alternative embodiments, protection from degradation can be obtained by minimizing all reactions that cause degradation. For instance, selecting a pH in aqueous solutions where the rates of acidic, alkaline, or neutral reactions are minimized can significantly enhance stability. Employing inert gases can further help mitigate degradation, as can dissolving the chemicals in solvents with low reactivity, such as DMSO or THF. Adding "scavengers" that react with oxygen or absorb harmful substances can help preserve the integrity of the chemicals. Moreover, encapsulating the chemicals in polymers, sugars, or other materials that form a gas-tight layer can reduce exposure to moisture and oxygen, while also allowing them to dissolve into the solution used in the vial. In one or more specific examples, a DMSO:isopropanol mixture, and an aqueous mixture of HEPES adjusted to pH 6.8 in proportions of 9:9:2 may be used. This combination has proven effective in maintaining the stability of sensitive compounds.

In one or more embodiments, which may be combined with different environments within the composition chamber 121, it may be possible to protect photosensitive molecules of the composition 122 from light by utilizing non-translucent plastics for the walls of the internal standard chamber.

The composition chamber 121 may further comprise an abrasive element 124 stored therein. The abrasive element 124 may be arranged such that, when the driving member 107 pushes the predetermined volume of the fluid sample and the composition 122 through the outlet 120, the abrasive element 124 is also pushed through the outlet 120. The abrasive element 124 may be configured to enhance mechanical mixing of the predetermined volume of fluid and the composition 122 in a vessel into which they are pushed upon leaving the outlet 120. For example, the abrasive element 124 may be an abrasive ball or other object which, when shaken in a vessel, serves to provide for mechanical mixing. The enhanced mixing may promote effective protein precipitation through fine grinding of the precipitate. In one or more embodiments, a plurality of abrasive elements 124 may be contained within the composition chamber 121 or one or more additional abrasive elements may be contained within a vessel into which the predetermined volume of sample is to be dispensed.

The abrasive element may be, for example, a bead, ball, gem, bullet, mixer and/or wire, for example a metal ball or otherwise, such as a stainless steel ball, or any related material. For the avoidance of doubt, when referring to "the composition 122" this refers to any composition 122 suitable for being stored within the composition chamber 121 and is not necessarily limited to a matrix composition 122, although any aspects of the composition 122 as defined herein may be incorporated into the matrix composition 122 defined above.

By the composition chamber 122 comprising abrasive elements, mixing of the sample fluid, composition 122 and/or analysis/extraction solution is improved due to enhanced mixing when agitating (e.g. shaking) the sample apparatus following introduction of the sample. This may reduce sample preparation time, enhance homogeneous precipitation of proteins and provide quantitative release of the internal standard from the composition 122 during mixing, while having a negligible negative impact on the analysis results. Stainless steel balls may be particularly advantageous to provide higher yields and reduced variation in measured results.

When the matrix composition 122 comprises polymers, such as non-woven polypropylene, the inventors have found that the additional weight provided by the abrasive element or elements, in particular stainless steel balls, prove highly effective in achieving quick preparation time and enhanced precipitation.

The mass of the abrasive element comprised within the composition chamber 121, or incorporated into the interior of the sample vessel, may be from about 50 mg to about 500 mg, such as about 100 mg to about 200 mg, for example about 150 mg to about 250 mg.

The composition chamber 121 or the interior of the sample vessel may comprise from about 1 to about 20 abrasive elements, such as from about 2 to about 10 abrasive elements, such as from about 3 to about 7 abrasive elements.

The abrasive elements may have a diameter of from about 0.1 mm to about 1 cm, such as from about 0.5 mm to about 5 mm, such as from about 0.8 to about 1.2 mm.

Preferably, the composition chamber 121, or the interior of the sample vessel, comprises one or more abrasive elements and a cosolvent as defined above. The combination of beads and solvent may prove particularly advantageous in protein precipitation from the analysis/extraction solution, leading to significantly improved accuracy of the analysis results.

Furthermore, when the composition chamber 121 or the interior of the sample vessel comprises abrasive elements, significantly better results (i.e. improved precipitation, higher yields and reduced variation in measured results) may be obtained when the apparatus containing steel balls is shaken manually, compared to shaking the apparatus without balls in a laboratory machine, therefore facilitating use of the apparatus by a user.

In one or more examples, the abrasive element 124 may be arranged within the composition chamber 121 between the composition 122 and the frangible base such that the composition 122 is pushed out of the outlet by the engagement rod 107 before the abrasive element 124. For example, the abrasive element 124 may be arranged above the composition 122 such that the driving member 107 or the broken frangible base 104 contacts the abrasive element 104 upon breaking the frangible base 104 and the driving member 107 applies force to the composition 122 via the abrasive element 124. This may reduce any loss of the composition which may otherwise occur by rubbing of the composition 122 onto the driving member 107. Since the abrasive element 124 is configured to enter a subsequent vessel, by way of its being forced out of the outlet 120 anyway, this may ensure, or increase the likelihood, that all of the composition 122 leaves the outlet.

Figure 4 shows an example according to the present disclosure of a composition dispensing device 200 comprising a composition chamber 201. The composition chamber 201 is sealed at a first end by a first frangible seal 202 and at a second end by a second frangible seal 203. The composition chamber 201 comprises: a composition 204; and an abrasive element 205 stored therein, wherein the abrasive element 205 is arranged proximal to the first end of the composition chamber 200 and the composition 204 is arranged proximal to the second end of the composition chamber 200 such that a driving member which breaks the first frangible seal and moves through the composition chamber 201 contacts the abrasive element 205 and, upon the application of further force by a driving member, the abrasive member 205 contacts the composition 204 such that the abrasive member 205 and the composition 204 are forced out of the composition chamber 201. The composition dispensing device 200 may be configured to be coupled to a sample vessel at its second end such that a driving member can break the first frangible seal 202 and force the composition 204 followed by the abrasive element 205 through the second frangible seal 203. It will be appreciated that this composition dispensing device 200 is similar to the composition chamber 121 comprising the abrasive element 124 described above with respect to the sample collection device 100 but without the parts of the disclosure which provide for the isolation of a predetermined volume of sample fluid. It will, then, be further appreciated that any of the options described above with respect to the configuration of the composition chamber 121, its frangible barrier 123, the composition 122 and the abrasive element 124 may equally be applied to the composition dispensing device 200 described here and depicted by way of example in Figure 4. For example, the composition 204 may comprise one or more of a fluid processing reagent and an internal standard. The first and second frangible barriers may comprise the properties of the frangible barrier 123 discussed with respect to the sample collection device 100.

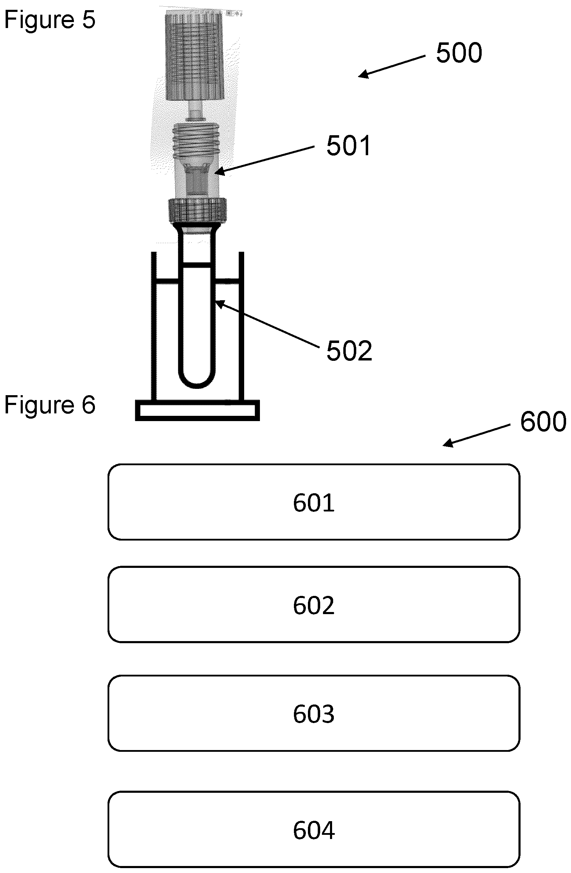

Figure 5 shows an example kit of parts 500 comprising the sample collection device 501 of any of the embodiments described above and a sample vessel 502 into which the predetermined volume of fluid can be dispensed and stored. The kit may further comprise a lancet. The lancet may be useable by a user to prick their body in order to release blood which can then be transferred into the sample collection device. For the avoidance of doubt, by the term "lancet" we refer to a skin-puncturing device which comprises needles or narrow, sharp blades that poke a small hole in the skin obtain capillary blood. In the context of the present invention the lancet is used to extract blood of the subject through the skin for the sampler to draw up. The sample vessel of the kit of parts may comprise one or more abrasive elements, such as beads, balls, gems, bullets, mixers and/or wires. The abrasive element or elements may be provided within the interior of the sample vessel, such as residing within an analysis/extraction solution.

Figure 6 shows an example method 600 according to the present disclosure. The method 600 comprises receiving 601 fluid within a well of a sample collection device, the well comprising a frangible base; moving 602 a driving member into the well, wherein the driving member is configured to isolate the predetermined volume of the fluid; applying 603 a predetermined force to the driving member to break the frangible base of the well; and moving 604 the predetermined volume of fluid out of an outlet of the sample collection device by pushing the driving member to push the predetermined volume of fluid through the outlet.

It will be appreciated that the features and embodiments disclosed herein above may be combined together in any manner except for where to do so would be explicitly against the teachings of the present disclosure. By way of non-limiting example, the implementation of the composition chamber and the engagement rod may both be incorporated into a single embodiment, or only one of these may form an embodiment. In other examples, embodiments may be envisioned by the skilled person wherein the composition chamber comprising an abrasive element are provided in the same embodiment where a top part body is transparent or translucent. Every possible combination or permutation of features has not been described explicitly for brevity and so as to avoid obfuscating the benefits of each of the features disclosed here. Further, while some features are listed in seemingly separate embodiments, this does not imply that some features may not be advantageously synergistically combined in order to provide for a contribution which is greater than the sum of its parts. Similarly, while some features are depicted together, embodiments are still envisioned wherein only one feature, or a subset of these features, is needed to implement a device according to the present disclosure.

The following outlines a proof of concept study performed using a sample collection device according to one embodiment of the present disclosure. Objective: To demonstrate that the functionality works as intended by measurement of the extracted sample weight (volume) when adding sample in three different values.

Method: The sampler was 3D-printed using 1.75 mm Bambu Lab PETG-HF filament on a Bambu Lab P1S printer. To prevent errors from water evaporation in blood, a simulated blood sample was prepared using olive oil (90%) and hexylene glycol (10%) (Sigma-Aldrich), coloured with methylene blue (0.05%) (Sigma-Aldrich). Predefined total sample volumes (80, 120, and 180 µL) of the coloured oil were added (weight gain measured) to individual proof-of-concept devices, with four replicates per volume (n=12). The sampler's weight was recorded before and after extraction. The extracted volume was determined by measuring the absorbed solvent on laboratory wipers. The residual oil in the sampler was also weighed to verify accuracy.

The samples were pipetted with a predefined volume, independent of the padded sample amount. The small device extracted an average volume of 42.7 µL with a standard deviation (SD) of 2.3 µL. Only one sample deviated by more than 10% (11.7%), which remained within the proof-of-concept threshold of 15% for the difference between the added and extracted sample weights. These results indicate that the prototype performed well within a predefined benchmark defined as an accuracy of ± 15 %. It was also confirmed in the experimental results that the volume extracted was independent of the total sample volume received within the prototype well.

## Claims

1. A sample collection device, comprising:
a well configured to receive a fluid sample, the well comprising a frangible base;
a driving member configured to be moveable into the well and to isolate a predetermined volume of the fluid sample of that received within the well, the driving member further configured to move the predetermined volume of fluid sample out of an outlet of the sample collection device by breaking the frangible base of the well upon the application of a predetermined force and pushing the predetermined volume of the fluid sample through the outlet.

2. The sample collection device of claim 1 wherein:
the well comprises an inlet arranged opposite the frangible base;
the frangible base comprises an engagement rod extending from the frangible base towards the well inlet;
the driving member is configured to engage with the engagement rod such that, upon engagement of the driving member with the engagement rod, the predetermined volume of the fluid sample is isolated; and
the frangible base of the well is configured to be broken upon the application of the predetermined force to the driving member when the driving member is engaged with the engagement rod.

3. The sample collection device of claim 2 wherein the length of the engagement rod is such that, if a fluid collected within the well encapsulates a tip of the engagement rod, then there is sufficient fluid within the well to isolate the predetermined volume of fluid.

4. The sample collection device of any preceding claim further comprising a composition chamber defined between the frangible base and the outlet of the sample collection device, wherein the composition chamber comprises a composition sealed therein, wherein the composition comprises one or both of: a fluid processing reagent and an internal standard and wherein the driving member is configured to push both the predetermined volume of the fluid sample and the composition through the composition chamber and out of the outlet.

5. The sample collection device of claim 4 further comprising an abrasive element stored within the composition chamber wherein the abrasive element is positioned such that when the driving member pushes the predetermined volume of the fluid sample and the composition through the outlet, the abrasive element is also pushed through the outlet and wherein the abrasive element is configured, to enhance mechanical mixing of the predetermined volume of fluid and the composition in a vessel into which they are pushed upon leaving the outlet.

6. The sample collection device of claim 5 wherein the abrasive element is arranged within the composition chamber between the composition and the frangible base such that the composition is pushed out of the outlet by the engagement rod before the abrasive element.

7. The sample collection device of any preceding claim comprising an upper cap configured to close over the well inlet, wherein the upper cap comprises the driving member.

8. The sample collection device of claim 7 wherein the upper cap comprises an upper cap body which is configured to be gripped by a user and wherein the upper cap is formed at least partially of material that allows inspection through the upper cap.

9. The sample collection device of claim 7 or claim 8 wherein the upper cap comprises an upper cap body that is configured to be gripped by a use and wherein the upper cap body is rotationally isolated from the driving member such that rotation of the upper cap body does not result in rotation of the driving member.

10. The sample collection device of any preceding claim wherein the well comprises a first volume having a first cross-sectional width and a second cross-sectional width, wherein the first cross-sectional width is smaller than the second cross-sectional width and wherein, when the driving member isolates the predetermined volume of fluid, the predetermined volume of fluid is contained within the first volume of the well.

11. The sample collection device of any preceding claim wherein the well comprises a channel arranged such that, as the driving member is moved through the well to isolate the predetermined volume of fluid, any excess fluid that would exceed the predetermined volume is displaced through the channel.

12. The sample collection device of claim 11 when dependent on claim 10, wherein the channel extends from an upper portion of the first volume to the second volume.

13. The sample collection device of any preceding claim wherein the predetermined force required to break the frangible base is greater than a force required to move the driving member through the well into a position which isolates the predetermined volume of fluid.

14. A kit of parts comprising the sample collection device of any preceding claim and a sample vessel in which the predetermined volume of fluid can be stored after it has moved out of the outlet of the sample collection device.

15. A method of isolating and outputting a predetermined volume of a fluid, the method comprising:
receiving fluid within a well of a sample collection device, the well comprising a frangible base;
moving a driving member into the well, wherein the driving member is configured to isolate the predetermined volume of the fluid;
applying a predetermined force to the driving member to break the frangible base of the well;
moving the predetermined volume of fluid out of an outlet of the sample collection device by pushing the driving member to push the predetermined volume of fluid through the outlet.
